**Europäisches Patentamt**

**European Patent Office**

**Office européen des brevets**

(19)

(11) Veröffentlichungsnummer: **0 281 780 B1**

(12) # EUROPÄISCHE PATENTSCHRIFT

(45) Veröffentlichungstag der Patentschrift:
06.03.91 Patentblatt 91/10

(51) Int. Cl.$^5$: **G01N 27/22**

(21) Anmeldenummer: **88101961.6**

(22) Anmeldetag: **10.02.88**

(54) **Verfahren und Einrichtung zum Bestimmen der volumetrischen Zusammensetzung einer Mehrkomponentenströmung.**

(30) Priorität: **23.02.87 DE 3705737**

(43) Veröffentlichungstag der Anmeldung:
**14.09.88 Patentblatt 88/37**

(45) Bekanntmachung des Hinweises auf die Patenterteilung:
**06.03.91 Patentblatt 91/10**

(84) Benannte Vertragsstaaten:
**DE GB**

(56) Entgegenhaltungen:
**DE-A- 1 498 797**
**DE-A- 3 518 186**

(73) Patentinhaber: **Siemens Aktiengesellschaft**
**Wittelsbacherplatz 2**
**W-8000 München 2 (DE)**

(72) Erfinder: **Kefer, Volker**
**Oppelner Strasse 7**
**W-8520 Erlangen (DE)**
Erfinder: **Krätzer, Werner**
**Auf der Höhe 33**
**W-8551 Röttenbach (DE)**

## Beschreibung

Die Erfindung betrifft ein Verfahren zum Bestimmen der volumetrischen Zusammensetzung einer Mehrkomponentenströmung in einem Rohr.

Gemische aus mehreren Komponenten werden in unterschiedlichen technischen Bereichen durch Rohrleitungen transportiert. Ein häufig vorhandenes inhomogenes Gemisch aus drei Komponenten besteht aus Öl, Wasser und einem Gas. Dieses Gemisch tritt insbesondere bei der Ausbeutung von Erdölfeldern auf und wird dabei durch Rohrleitungen geführt.

Aus verschiedenen Gründen ist es notwendig, die volumetrischen Anteile der einzelnen Komponenten einer Mehrkomponentenströmung zu bestimmen. Bei der Ausbeutung von Erdölfeldern ist der Ölanteil des Gemisches von besonderem Interesse. Aber auch der volumetrische Anteil von gleichzeitig erschlossenem Erdgas soll bestimmt werden.

Bisher bekannte Verfahren zum Bestimmen der volumetrischen Zusammensetzung erfordern eine Probennahme aus dem Rohr, in dem das Gemisch strömt. Anschließend werden die Komponenten separiert und getrennt deren Volumenstrom gemessen. Derartige Meßverfahren sind technisch aufwendig und nehmen viel Zeit in Anspruch. Folglich ist eine kontinuierliche Überwachung der volumetrischen Zusammensetzung nicht realisierbar. Bekannte Meßverfahren beschränken sich auf Messungen in regelmäßigen Abständen.

Der Erfindung liegt die Aufgabe zugrunde, ein Verfahren und eine Einrichtung zu entwickeln, die ohne Probennahme und ohne Komponentenseparation direkt im durchströmten Rohr eine kontinuierliche, zuverlässige Messung der volumetrischen Zusammensetzung der strömenden Mehrkomponentenmischung, die beispielsweise Öl, Gas und Wasser enthält, gewährleistet.

Die Aufgabe wird erfindungsgemäß dadurch gelöst,
daß innerhalb eines Meßzyklus bei einer ersten Messung die Kapazität eines im Rohr in der Strömung angeordneten Kondensators, dessen elektrisches Feld durch eine erste Prüfspannung festgelegt ist, gemessen wird, wobei die Kapazität des Kondensators über die Dielektrizitätszahl und die Polarisation des strömenden Mediums von dessen Zusammensetzung abhängt,
daß bei weiteren Messungen die Kapazität des Kondensators bei elektrischen Feldern, die durch weitere Prüfspannungen festgelegt sind und die eine Polarisationsänderung bei einem Teil der Komponenten zur Folge haben, gemessen wird und
daß aus den gemessenen Kapazitätswerten und aus einmal zu bestimmenden Kapazitäten des Kondensators, die bei Füllung mit jeweils nur einer der vorhandenen Komponenten und bei jeweils einer der gewählten Prüfspannungen gemessen werden, die volumetrischen Anteile der Komponenten in der Strömung bestimmt werden.

Die Kapazität eines Kondensators wird außer durch seine Geometrie durch die Dielektrizitätszahl des sich zwischen den Kondensatorplatten befindenden Mediums bestimmt. Die Dielektrizitätszahl hängt von der Polarisierbarkeit des Mediums im elektrischen Feld des Kondensators ab. Mit der Erfindung wird ausgenützt, daß verschiedenartige Stoffe im gleich starken elektrischen Feld unterschiedlich polarisierbar sind. Besonders deutlich ist der Unterschied beim Vergleich der Polarisierbarkeit von Wasser und Öl.

Aus der DE-AS- 4 98 797 ist ein Verfahren zum Messen des Staubgehaltes der Luft bekannt. Dabei wird die Luft durch einen Kondensator geleitet und ein Spannungsabfall über einen ohmschen Widerstand im Kondensatorstromkreis wird gemessen. Dabei wird ein Verschiebungsstrom als Hinweis auf den Staubgehalt bestimmt. Das bekannte Verfahren ist nur für die Messung von Aerosolen in einem gasförmigen Träger geeignet. Dabei handelt es sich nicht um eine Mehrkomponentenströmung.

Eine Vorrichtung gemäß der DE-A- 35 8 86 dient zum Bestimmen des Wasseranteiles in einem Öltank. Die Messung erfolgt am ruhenden Medium. Die Messung wird mit einem Sender und einem Empfänger durchgeführt, wobei ein Signal das Medium durchdringt und Änderungen der Frequenz und der Phase des Signales den Wasseranteil angeben. Ein solches Verfahren wird nicht eingesetzt zum Bestimmen der volumetrischen Zusammensetzung einer Mehrkomponentenströmung in einem Rohr.

Wassermoleküle sind elektrische Dipole, die in einem elektrischen Feld ausgerichtet werden. Die Polarisierung von Ölmolekülen beruht hingegen auf einem anderen Effekt. In einem Ölmolekül werden, wenn es sich in einem elektrischen Feld befindet, die elektrisch negativen Elektronen relativ zum positiven Atomkern verschoben. Die Verschiebungspolarisation des Öles benötigt eine weit geringere Feldenergie als die Orientierungspolarisation des Wassers.

Erfindungsgemäß wird die elektrische Feldstärke des Kondensators mittels der angelegten Spannung zunächst so eingestellt, daß das vorhandene Öl vollständig und das Wasser nur teilweise polarisiert ist. Dann wird eine Kapazitätsmessung am Kondensator durchgeführt. Der Kapazitätswert der ersten Messung setzt sich folgendermaßen zusammen :

$$C_1 = A_W \cdot C_{W1} + A_{\ddot{O}} \cdot C_{\ddot{O}1} + A_G \cdot C_{G1}$$

Dabei sind $C_{W1}$, $C_{\ddot{O}1}$ und $C_{G1}$ Kapazitäten des Kondensators, die zuvor mit der gewählten Feldstärke gemessen wurden, wobei der Kondensator jeweils vollständig mit nur einer der Komponenten, nämlich Wasser, Öl oder Gas gefüllt war. Diese Kapazitätswerte sind nach einmaliger Bestimmung als Systemkonstanten bei der Durchführung des Verfahrens stets einzusetzen.

$A_W$, $A_Ö$ und $A_G$ in der genannten Gleichung sind die Volumenanteile der Komponenten Wasser, Öl und Gas, die bestimmt werden sollen.

Dazu ist ein Gleichungssystem aus drei linear unabhängigen Gleichungen erforderlich. Die zweite Gleichung ist bereits dadurch gegeben, daß die Summe der Volumenanteile den Wert 1 ergeben muß.

$$A_w + A_Ö + A_G = 1$$

Die dritte Gleichung ergibt sich dadurch, daß erfindungsgemäß eine zweite Kapazitätsmessung in der Mehrkomponentenströmung durchgeführt wird. Das wesentliche Merkmal der Erfindung ist dabei darin zu sehen, daß die Feldstärke im Kondensator mittels der angelegten Spannung für diese zweite Messung soweit angehoben wird, daß der Polarisierungsgrad des Wassers gegenüber der ersten Messung höher liegt. Der Polarisierungsgrad von Öl bleibt hingegen konstant, da er bereits bei der ersten Messung seinen maximalen Wert erreicht hatte. Gas ist überhaupt nicht polarisierbar, so daß auch hier zwischen den beiden Messungen kein Unterschied besteht. Der zweite bei größerer Feldstärke gemessene Kapazitätswert setzt sich wie folgt zusammen :

$$C_2 = A_W \cdot C_{W2} + A_Ö \cdot C_{Ö2} + A_G \cdot C_{G2}$$

Da die angelegten elektrischen Felder, wie zuvor beschrieben, ausgewählt wurden, entsprechen sich $C_{Ö1}$ und $C_{Ö2}$ sowie $C_{G1}$ und $C_{G2}$. Nur $C_{W1}$ unterscheidet sich von $C_{W2}$, da die Feldstärke von der ersten zur zweiten Messung so geändert wurde, daß sich nur der Polarisierungsgrad des Wassers ändert. Der Wert von $C_{W2}$ muß bei vollständig mit Wasser gefülltem Kondensator und bei der für die zweite Messung gewählten Feldstärke vor dem Einsatz des Kondensators bestimmt werden und stellt genauso wie $C_{W1}$, $C_{Ö1}$ und $C_{G1}$ eine Systemkonstante dar.

Mit dem erfindungsgemäßen Verfahren wird der Vorteil erzielt, daß auf einfache Weise drei linear unabhängige Gleichungen für die Volumenanteile von Wasser, Öl und Gas, aufzustellen sind Daraus ergeben sich sofort die gesuchten Volumenanteile.

Falls eine Mischung aus mehr als drei Komponenten auf ihre volumetrische Zusammensetzung hin überprüft werden soll, ist für jede weitere Komponente eine zusätzliche Kapazitätsmessung bei anderer Feldstärke durchzuführen. Dabei werden unterschiedliche Gradienten des Polarisierungsgrades der Komponenten bei ansteigender Feldstärke im Kondensator berücksichtigt.

Eine Einrichtung zur Durchführung des erfindungsgemäßen Verfahrens weist einen Kondensator auf, der im von einer Mehrkomponentenströmung durchströmten Rohr angeordnet ist. Der Kondensator ist mit einem Kapazitätsmeßgerät verbunden. Ein wesentliches Merkmal der Erfindung ist, daß die am Kondensator anliegende Spannung veränderbar ist.

Durch die Veränderung der Spannung ist das elektrische Feld innerhalb des Kondensators und damit der Polarisierungsgrad der im Rohr strömenden Komponenten veränderbar. Die am Kapazitätsmeßgerät angezeigten Werte sind gemäß dem erfindungsgemäßen Verfahren miteinander verknüpfbar, so daß man die volumetrischen Anteile der in der Strömung vorhandenen Komponenten erhält.

Am Kondensator liegt in der Regel eine Wechselspannung an. Dabei ist, damit schnell eintretende Änderungen in der Mehrkomponentenströmung zu erfassen sind, eine hochfrequente Wechselspannung erforderlich, deren Frequenz beispielsweise 1 MHz ist.

Zur Variierung des elektrischen Feldes im Kondensator ist beispielsweise mit dem Kondensator in Serie mindestens ein ohmscher Widerstand geschaltet. Jeder dieser Widerstände ist einzeln durch niederohmige mit Schaltern versehene Leitungen überbrückt. Dadurch sind die Widerstände einzeln in den Stromkreis des Kondensators einschaltbar. Mit dieser Widerstandsanordnung wird der Vorteil erzielt, daß eine begrenzte Anzahl von zuvor festgelegten Spannungswerten am Kondensator wahlweise sehr exakt einstellbar sind. Bei einer Strömung im Rohr, die aus drei Komponenten besteht, genügt ein einziger mit dem Kondensator in Serie geschalteter überbrückbarer ohmscher Widerstand.

Mit der geschilderten Einrichtung wird der Vorteil erzielt, daß das erfindungsgemäße Verfahren zum Bestimmen der volumetrischen Zusammensetzung einer Mehrkomponentenströmung mit einfachen störunanfälligen apparativen Mitteln durchzuführen ist.

Aus den mit dem Kapazitätsmeßgerät bestimmten Kapazitätswerten sind die Volumenanteile der Strömungskomponenten zu bestimmen.

Mit der Erfindung wird der Vorteil erzielt, daß nach wenigen einmal durchzuführenden Kalibriermessungen die volumetrische Zusammensetzung einer Mehrkomponentenströmung in einem Rohr ohne Probennahme direkt im verschlossenen Rohr mit einfachen apparativen Mitteln schnell und zuverlässig zu bestimmen ist.

Das erfindungsgemäße Verfahren und die Einrichtung sind in verschiedenartigen Industriezweigen vorteilhaft einsetzbar. Beispielsweise lassen sich bei der Erdölförderung die Volumenanteile von Erdöl, Gas und Wasser im geförderten Gut zuverlässig bestimmen.

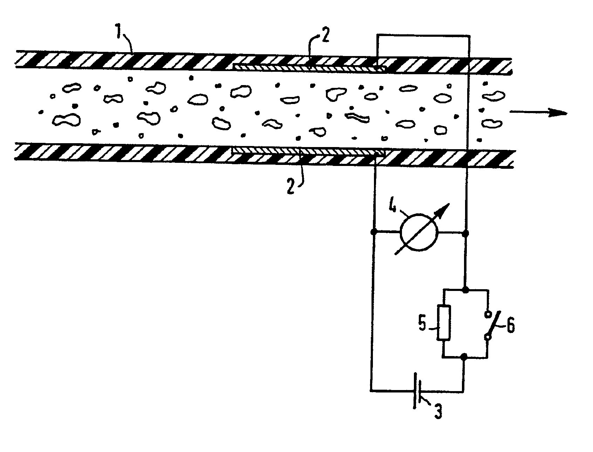

Die Erfindung wird anhand der Zeichnung näher erläutert :
Die Zeichnung zeigt eine Einrichtung zum Bestimmen der volumetrischen Zusammensetzung einer Mehrkomponentenströmung.

Ein Rohr 1 wird von einem inhomogenen Medium, das aus drei Komponenten besteht, durchströmt. Derartige inhomogene Dreikomponentenströmungen treten bei der Erdölförderung auf. Die drei Komponenten sind dabei Öl, Wasser und Gas. Zum Bestimmen der volumetrischen Zusammensetzung ist im Rohr 1 ein Kondensator 2 angeordnet. Das inhomogene Medium durchströmt den Kondensator 2 und wirkt dabei als Dielektrikum. Am Kondensator 2 liegt eine Spannungsquelle 3 an. Zum Bestimmen der Kapazität des Kondensators 2, die außer von dessen Geometrie und von der anliegenden Spannung auch vom Dielektrikum abhängt, ist der Kondensator 2 mit einem Kapazitätsmeßgerät 4 verbunden. Die Spannungsquelle 3 liefert eine konstante Spannung, so daß das elektrische Feld im Kondensator 2 konstant bleibt. Die Spannung und damit das elektrische Feld werden für eine erste Messung so gewählt, daß das leicht zu polarisierende Öl im Rohr 1 vollständig polarisiert ist, während das schwer zu polarisierende Wasser im Rohr 1 nur zum geringen Teil polarisiert ist. Für eine zweite Messung wird die Spannung am Kondensator 2 und damit die Feldstärke des elektrischen Feldes soweit angehoben, daß nunmehr auch das Wasser stärker als bei der ersten Messung polarisiert ist. Aus den beiden Kapazitätswerten erhält man zusammen mit den Kapazitätswerten des vollständig mit jeweils einer Komponente der Strömung gefüllten Kondensators 2, die volumetrischen Anteile von Öl, Wasser und Gas. Die Kapazitäten des Kondensators 2 bei Füllung mit jeweils einer Komponente werden für beide anzulegenden Spannungen vor dem ersten Einsatz der Einrichtung durch einmaliges Messen bestimmt. Sie stehen für alle Messungen zur Verfügung. Falls die beiden Spannungswerte so gewählt sind, daß nur Auswirkungen auf die Polarisation einer Komponente gegeben sind, reichen vier Kalibriermessungen aus. Damit zwei auf einfache Weise reproduzierbare Spannungen wahlweise am Kondensator 2 anlegbar sind, ist mit dem Kondensator 2 in Serie ein ohmscher Widerstand 5 angeordnet, der durch eine niederohmige mit einem Schalter 6 versehene Leitung überbrückt ist. Für die erste Messung ist der Schalter 6 offen. Am Kondensator 2 liegt dann eine durch den Widerstand 5 festgelegte Teilspannung an. Für die zweite Messung wird der Schalter 6 geschlossen, so daß die höhere Spannung der Spannungsquelle 3 am Kondensator 2 anliegt. Die Spannungsquelle 3 und der ohmsche Widerstand 5 sind so bemessen, daß die beiden zur Verfügung gestellten Spannungen die jeweils gewünschte Polarisierung von Wasser und Öl im Rohr 1 bewirken. Aus den Meßwerten des Kapazitätsmeßgerätes 4 ist mit der geschilderten Einrichtung bei Anwendung des erfindungsgemäßen Verfahrens die volumetrische Zusammensetzung einer Mehrkomponentenströmung in einem geschlossenen Rohr, beispielsweise in einem Rechner, zuverlässig zu bestimmen.

Statt eines Kondensators 2 mit veränderbarer Prüfspannung können auch zwei Kondensatoren mit gleichbleibender unterschiedlicher Feldstärke verwendet werden.

## Ansprüche

1. Verfahren zum Bestimmen der volumetrischen Zusammensetzung einer Mehrkomponentenströmung in einem Rohr (1), **dadurch gekennzeichnet,** daß innerhalb eines Meßzyklus bei einer ersten Messung die Kapazität eines im Rohr (1) in der Strömung angeordneten Kondensators (2), dessen elektrisches Feld durch eine erste Prüfspannung festgelegt ist, gemessen wird, wobei die Kapazität des Kondensators (2) über die Dielektrizitätszahl und die Polarisation des strömenden Mediums von dessen Zusammensetzung abhängt, daß bei weiteren Messungen die Kapazität des Kondensators (2) bei elektrischen Feldern, die durch weitere Prüfspannungen festgelegt sind, und die eine Polarisationsänderung bei einem Teil der Komponenten zur Folge haben, gemessen wird und daß aus den gemessenen Kapazitätswerten und aus einmal zu bestimmenden Kapazitäten des Kondensators (2), die bei Füllung mit jeweils nur einer der vorhandenen Komponenten und bei jeweils einer der gewählten Prüfspannungen gemessen werden, die volumetrischen Anteile der Komponenten in der Strömung bestimmt werden.

2. Einrichtung zur urchführung des Verfahrens nach Anspruch 1, **dadurch gekennzeichnet**, daß im von einer Mehrkomponentenströmung durchströmten Rohr (1) ein Kondensator (2) angeordnet ist, der mit einem Kapazitätsmeßgerät (4) verbunden ist, und daß die am Kondensator (2) anliegende Spannung zur Bildung der ersten und der weiteren Prüfspannungen veränderbar ist.

3. Einrichtung nach Anspruch 2, **dadurch gekennzeichnet**, daß der Kondensator (2) mit einer Spannungsquelle (3), die eine konstante Spannung liefert, verbunden ist, daß mit dem Kondensator (2) in Serie mindestens ein ohmscher Widerstand (5) geschaltet ist und daß die ohmschen Widerstände (5) einzeln durch niederohmige mit Schaltern (6) versehene Leitungen überbrückt sind, so daß die ohmschen Widerstände (5) einzeln in den Stromkreis des Kondensators (2) zur Bildung der ersten und der weiteren Prüfspannungen einschaltbar sind.

4. Einrichtung nach Anspruch 2, **dadurch gekennzeichnet**, daß der Kondensator (2) mit einer Spannungsquelle (3), die eine Wechselspannung liefert, verbunden ist.

## Claims

1. Process for determining the volumetric composition of a multi-component flux in a conduit (1), characterised in that

in a measuring cycle in a first measurement the capacitance of a capacitor (2) is measured, which capacitor is arranged in the flux in the conduit (1), the electric field of which is determined by a first test voltage, whereby the capacitance of the capacitor (2) depends, through the dielectric constant and the polarisation of the flowing medium, on the composition thereof,

in that in the course of further measurements the capacitance of the capacitor (2) in electric fields, which are determined by further test voltages, and which give rise to a polarisation change in one part of the components, is measured and

in that from the measured capacitance values and from capacitances of the capacitor (2) to be determined once, which are measured when filling with respectively only one of the components present and with respectively one of the selected test voltages, the volumetric portions of the components are determined in the flux.

2. Device for carrying out the process according to claim 1, characterised in that in a conduit (1) through which flows a multi-component flux there is arranged a capacitor (2) which is connected to a capacitance measuring device (4), and in that the voltage at the capacitor (2) can be changed to form the first and the further test voltages.

3. Device according to claim 2, characterised in that the capacitor (2) is connected to a voltage source (3) which yields a constant voltage, in that at least one ohmic resistance (5) is connected in series with the capacitor (2) and in that the ohmic resistances (5) are bridged individually by low ohmic leads provided with switches (6), so that the ohmic resistances (5) can be individually connected into the circuit of the capacitor (2) to form the first and the further test voltages.

4. Device according to claim 2, characterised in that the capacitor (2) is connected to a voltage source (3) which yields an alternating voltage.

## Revendications

1. Procédé de détermination de la composition volumétrique d'un courant à plusieurs constituants dans un tuyau (1), caractérisé en ce qu'il consiste

à mesurer dans un cycle de mesure, lors d'une première mesure, la capacité d'un condensateur (2) qui est disposé dans le courant qui s'écoule dans le tube (1) et dont le champ électrique est déterminé par une première tension d'essai, la capacité du condensateur (2) dépendant, par l'intermédiaire de la constante diélectrique et de la polarisation du milieu en écoulement, de la composition de celui-ci,

à mesurer, lors de mesures ultérieures, la capacité du condensateur (2) pour des champs électriques qui sont fixés par d'autres tensions d'essai et qui se traduisent par une variation de la polarisation d'une partie des constituants, et

à déterminer à partir des valeurs de la capacité mesurées et des capacités du condensateur (2), qui ont été déterminées auparavant et qui sont mesurées pour un remplissage par l'un seulement des constituants présents et pour l'une des tensions d'essai choisies, les proportions volumétriques des constituants dans le courant.

2. Dispositif pour la mise en oeuvre du procédé selon la revendication 1, caractérisé en ce que dans le tuyau (1) dans lequel passe un courant à plusieurs constituants est monté un condensateur (2) qui est relié à un appareil de mesure de la capacité (4) et en ce que la tension aux bornes du condensateur (2) peut être modifiée pour former la première tension d'essai et les tensions d'essai suivantes.

3. Dispositif selon la revendication 2, caractérisé en ce que le condensateur (2) est relié à une source de tension (3) qui fournit une tension constante, en ce qu'est montée en série avec le condensateur (2) au moins une résistance ohmique (5) et en ce que les résistances ohmiques (5) sont shuntées individuellement par des conducteurs de faible ohmicité et munis d'interrupteurs (6) de sorte que les résistances ohmiques (5) peuvent être mises individuellement dans le circuit du condensateur (2) pour former la première tension d'essai et les tensions d'essai suivantes.

4. Dispositif selon la revendication 2, caractérisé en ce que le condensateur (2) est relié à une source de tension (3) qui fournit une tension alternative.